# EUROPEAN PATENT SPECIFICATION

(11) **EP 3 881 855 B1**
(45) Date of publication and mention of the grant of the patent: **07.08.2024**
(21) Application number: 19928015.7
(22) Date of filing: 08.05.2019
(51) Int. Cl.: A61K 36/68, A61P 29/00, A61P 37/08, A61P 11/06, A23L 33/105

(54) **COMPOSITION FOR PREVENTING OR TREATING INFLAMMATION, ALLERGIES AND ASTHMA, CONTAINING VERONICASTRUM SIBIRICUM L. PENNELL AS ACTIVE INGREDIENT, AND USE THEREOF**
ZUSAMMENSETZUNG ZUR VORBEUGUNG ODER BEHANDLUNG VON ENTZÜNDUNGEN, ALLERGIEN UND ASTHMA MIT VERONICASTRUM SIBIRICUM L. PENNELL ALS WIRKSTOFF UND VERWENDUNG DAVON
COMPOSITION POUR PRÉVENIR OU TRAITER UNE INFLAMMATION, DES ALLERGIES ET DE L'ASTHME, CONTENANT VERONICASTRUM SIBIRICUM L. PENNELL EN TANT QUE PRINCIPE ACTIF, ET SON UTILISATION

(43) Date of publication of application: 22.09.2021
(73) Proprietor: KT&G Corporation, Daedeok-gu Daejeon 34337 (KR)
(72) Inventor: SHIN, Han-Jae, Daejeon 34128 (KR); JEH, Byong-Kwon, Daejeon 34128 (KR); LEE, Moon-Yong, Daejeon 34128 (KR); GWAK, Hyo-Min, Daejeon 34128 (KR); CHO, Sung-Eel, Daejeon 34128 (KR); KIM, Young-Sin, Daejeon 34128 (KR); LEE, Dong-Hun, Daejeon 34128 (KR); KIM, Chul-Young, Anyang-si Gyeonggi-do 14069 (KR)
(74) Representative: Ter Meer Steinmeister & Partner
(86) International application number: PCT/KR2019/005466
(87) International publication number: WO 2020/226204

(56) References cited:
- CN-A- 105 288 305
- JP-A- 2008 517 875
- KR-A- 20110 095 765
- KR-A- 20140 087 982
- KR-A- 20190 118 019
- KR-B1- 101 700 132
- KIM MYEONG IL ET AL: "Four New Acylated Iridoid Glycosides from the Aerial Part of Veronicastrum sibiricum and Their Antioxidant Response Element-Inducing Activity", CHEMISTRY & BIODIVERSITY, vol. 15, no. 1, 21 December 2017 (2017-12-21), CH, pages e1700447, XP055851800, ISSN: 1612-1872, Retrieved from the Internet <URL:https://api.wiley.com/onlinelibrary/tdm/v1/articles/10.1002%2Fcbdv.201700447> DOI: 10.1002/cbdv.201700447
- GAO W; ZHANG R; ZHANG I; TAKAISHI Y; DUAN H: "Immunosuppressive Diterpenes from Veronicastrum sibiricum", CHEM. PHARM. BULL., 2004, pages 136 - 137, XP055760219
- NAHM, D.-H.: "Present and future of allergen immunotherapy for allergic diseases", J KOREAN MED ASSOC, May 2015 (2015-05-01), pages 433 - 440, XP055760222, DOI: http://dx.doi.org/10.5124/jkma.2015.58.5.433
- WANG, Z.: "Anti-inflammation, Analgesic and Hemostatic Effects of Veronicastrum sibiricum", HERALD OF MEDICINE, 2017, pages 489 - 492, XP055760227, DOI: 10.3870/j.issn. 1004-0781 . 2017.05.00 7

## Description

### TECHNICAL FIELD

The present disclosure relates to a composition containing a Veronicastrum sibiricum L. Pannell extract as an active ingredient for preventing or treating allergies, and asthma, and a use of the composition.

### BACKGROUND ART

Generally, an inflammatory response is a defensive response of the human body associated with the regeneration of cells or tissues of the body when an invasion causes some organic change in the cells or tissues. Thus, a series of such responses include local blood vessels, various tissue cells in body fluids, immune-related cells, and the like. With recent developments in molecular biology, attempts have been made to understand the involvement of cytokines in inflammatory diseases at a molecular level, and factors affecting such diseases have been investigated one by one.

Allergic reactions may be classified into four categories, that is, types I, II, III, and IV, according to types of the allergic reaction. Alternatively, allergies of types I, II, and III may be referred to as immediate-type allergies, and allergies of type IV may be referred to as delayed-type allergies according to a period of time to onset from re-sensitization caused by allergens.

Among the allergies, the type 1 allergy is a reaction in which IgE antibodies are involved, and is referred to as anaphylaxis-type allergy, and examples of type 1 allergies include bronchial asthma, atopic diseases (e.g., dermatitis, gastroenteritis, etc.), allergic rhinitis such as pollinosis, allergic conjunctivitis, food allergies, and the like.

Asthma is a disease characterized by airway hyperresponsiveness to various stimuli, and clinical symptoms, for example, wheezing, dyspnea, and coughing caused by airway stenosis, may be naturally or reversibly recovered by treatment. Most asthma is allergic and is characterized by chronic airway inflammation and bronchial hyperresponsiveness (Minoguchi K and Adachi M. Pathophysiology of asthma. In: Cherniack NS, Altose MD, Homma I, editors. Rehabilitation of the patient with respiratory disease. New York: McGraw-Hill, 1999, pp97-104).

Asthma may be classified into extrinsic and intrinsic asthma, according to its causes. Extrinsic asthma is caused by the exposure to antigens. A positive reaction is shown in skin tests or bronchial provocation tests against the antigens, and extrinsic asthma generally occurs in young people. House dust and mites are main antigens, and pollen, animal epithelium, and fungi may also be antigens. Intrinsic asthma is caused or worsened by upper respiratory infection, exercise, emotional instability, cold weather, a change of humidity, and the like, and it is common to adult patients. In addition to extrinsic and intrinsic asthma, there may be exercise-induced asthma, occupational asthma, and the like.

Asthma has been regarded as a chronic inflammatory disorder because inflammatory cells are proliferated, differentiated, and activated by interleukin-4, 5, and 13 generated by T-helper2 (TH2) immune cells and then the inflammatory cells move and infiltrate into the airway and neighboring tissues thereof (Elias JA, et al., J. Clin. Invest., 111, pp291-297, 2003). The activated inflammatory cells such as eosinophils, mast cells, and alveolar macrophages in the bronchus of patients suffering from asthma release a variety of inflammatory mediators (e.g., cysteine leukotrienes, prostaglandins, etc.) and are involved in potent bronchial constriction (Maggi E., Immunotechnology, 3, pp233-244, 1998; Pawankar R., Curr.Opin. Allergy Clin. Immunol., 1, pp3-6, 2001; Barnes PJ, et al., Pharmacol Rev., 50, pp515-596, 1998).

Accordingly, the reproduction of various cytokines such as IL-4, IL-5, IL-13 and IgE, involved in inflammatory cell activation, and biosynthesis of cysteine leukotrienes released from the inflammatory cells such as eosinophils are the main causes of inflammation, allergic reactions, and asthma caused by inflammation and allergic reactions, and thus, much research has been conducted into the development of agents for inhibiting reproduction.

The present inventors have focused on the development of therapeutic agents using various resources, particularly, natural resources of which safety and efficacy are well known, as therapeutic agents using antibodies against a variety of cytokines and chemokines characterized in inflammation, allergies, and asthma.

KR10-2006-0125489 discloses effects of an extract of Veronica genus plants such as *Pseudolysimachion* ovtum, P. kiusianum, P. kiusianum var. diamanticum, P. kiusianum var. villosum, P. dahuricum, P. pyrethrinum, P. linarifolium, P. linarifolium var. villosulum, P. rotundum var. subintegrum, P. rotundum var. coreanum, P. insulare, P.undulata, and Veronica longifolia on treatment of inflammation, allergies, and asthma.

On the other hand, Veronicastrum sibiricum L. Pennell is in the genus Veronicastrum and is distinguished from plants in the genus Veronica such as Veronica longifolia, which grow in all areas of Korea, the Far East of Russia, Japan, northeastern regions of China, and the like, in that Veronicastrum sibiricum L. Pennell has whorled leaves and salverform corolla, not cup-shaped corolla, with four thin tips. (http://www.kbr.go.kr/home/rsc/rsc01002v.do?data_gbn_cd=BIO&ktsn_no=1200000633 71&menuKey=448).

However, none of the above literature discloses or teaches a medication for inflammation, allergies, and asthma that includes a Veronicastrum sibiricum L. Pennell extract (genus Veronicastrum) as an active ingredient.

Therefore, the present inventors have completed the present disclosure due to the confirmation, through various animal experiments, that a Veronicastrum sibiricum L. Pennell (genus Veronicastrum) extract exhibits better anti-inflammatory, anti-allergic and asthma-inhibitory activity than a Veronica longifolia (genus *Pseudolysimachion)* extract obtained via an existing method, the animal experiments being conducted using the Veronicastrum sibiricum L. Pennell (genus Veronicastrum) extract instead of the Veronica longifolia (genus *Pseudolysimachion)* extract disclosed in KR10-2006-0125489 and including: evaluation of inhibition of leukotriene production (Experimental Example 1); effects on the total number of cells in bronchoalveolar lavage (BAL) fluid by using male Balb/c mice (Experimental Example 2); effects on the number of neutrophil cells relative to the total number of cells in the BAL fluid (Experimental Example 3); effects on the number of Neutrophil+/Gr-1+ absolute cells in the BAL fluid (Experimental Example 4); effects on the number of CD11b+/Gr-1+ absolute cells from among lung cells (Experimental Example 5); effects on the number of CD4+/CD3+ absolute cells from among lung cells (Experimental Example 6); effects on the number of Macrophage+/CD1 1b+ absolute cells from among lung cells (Experimental Example 7); effects on the expression of inflammation factors in the BAL fluid (Experimental Example 8); and the like.

### DESCRIPTION OF EMBODIMENTS

### TECHNICAL PROBLEM

The present inventors have developed natural agents showing outstanding effects on the prevention or treatment of allergies, and asthma.

The present disclosure provides a pharmaceutical composition containing a Veronicastrum sibiricum L. Pennell (genus Veronicastrum) extract as an active ingredient for the prevention or treatment of allergies, and asthma

Also, the present disclosure provides a health functional food containing a Veronicastrum sibiricum L. Pennell (genus Veronicastrum) extract as an active ingredient for the prevention and improvement of allergies, and asthma

### SOLUTION TO PROBLEM

The present disclosure provides a pharmaceutical composition containing a Veronicastrum sibiricum L. Pennell (genus Veronicastrum) extract as an active ingredient for the prevention or treatment of allergies, and asthma

The term "Veronicastrum sibiricum L. Pennell" indicates the whole body, root, stem, or flower of Veronicastrum sibiricum L. Pennell from Korea, China, Russia, Japan, or other countries, preferably, Veronicastrum sibiricum L. Pennell that naturally grows or is cultivated in Korea, more preferably, Gyeonggi-do, Korea, most preferably, Ansan, Gyeonggi-do, Korea.

The term "Veronicastrum sibiricum L. Pennell extract" defined herein includes a crude extract, a polar soluble extract, or a non-polar soluble extract.

The term "crude extract" defined herein includes a solvent selected from water including purified water, a C₁-C₄ lower alcohol such as methanol, ethanol, or butanol, or a mixture thereof, preferably, methanol or a mixture of water and ethanol, more preferably, an extract soluble in 60 to 100% ethanol.

The term "non-polar soluble extract" defined herein includes an extract soluble in a solvent of hexane, methylene chloride, chloroform, or ethyl acetate, preferably, hexane, methylene chloride, or ethyl acetate, more preferably, ethyl acetate or methylene chloride.

The term "polar soluble extract" defined herein includes an extract soluble in a solvent selected from water, methanol, butanol, or a mixture thereof, preferably, water or butanol, more preferably, an extract soluble in butanol, except for the non-polar soluble extract.

The term "inflammation" defined herein (which is cited in the description but not part of the claimed subject-matter), may be any one inflammation selected from the group consisting of dermatitis, atopy, conjunctivitis, periodontitis, rhinitis, tympanitis, pharyngolaryngitis, tonsillitis, pneumonia, stomach ulcer, gastritis, a Crohn's disease, colitis, hemorrhoid, gout, ankylosing spondylitis, rheumatic fever, lupus, fibromyalgia, psoriatic arthritis, osteoarthritis, rheumatoid arthritis, periarthritis of shoulder, tendinitis, tendonitis, peritonitis, myositis, hepatitis, cystitis, nephritis, sjogren's syndrome, multiple sclerosis, and acute and chronic inflammatory diseases, but is not limited thereto.

The term "allergy" defined herein includes hypersensitivity, allergic rhinitis, asthma, allergic conjunctivitis, allergic dermatitis, atopic dermatitis, contact dermatitis, hives, insect allergy, food allergy or drug allergy, preferably, allergic rhinitis, asthma, allergic dermatitis, atopic dermatitis, contact dermatitis, hives, food allergy or drug allergy, more preferably, atopic dermatitis or contact dermatitis.

The term "asthma" defined herein includes bronchial asthma caused by a factor selected from the group consisting of house dust mites, pollen, animal hair or dandruff, cockroaches, food, drug, cold, cigarette smoke and indoor pollution, air pollution, food additives, physical activity such as exercise, climate change, yellow dust, and stress.

Hereinafter, the present disclosure will be described in more detail.

Extracts of the present disclosure may be obtained according to preparation methods below.

Hereinafter, for example, the present disclosure will be described in detail.

Crude extracts of the present disclosure may be prepared as follows. Dried Veronicastrum sibiricum L. Pennell is washed and sliced and mixed several times with a solvent selected from water including purified water, a C1-C4 lower alcohol such as methanol, ethanol, and butanol, or a mixture thereof, preferably, methanol or a mixture of water and ethanol, more preferably, 60 to 100% of ethanol. Then, an extract may be obtained by repeatedly performing ultra-sonication extraction, hot water extraction, roomtemperature extraction, or reflux extraction, preferably, ultra-sonication extraction, for 30 minutes to 48 hours, preferably, for one hour to 12 hours, at a temperature of between about 30 °C and about 150 °C, preferably, a temperature of between about a room temperature and about 100 °C, more preferably, a temperature of between about a room temperature and about 60 °C, about once to about 20 times, preferably, about twice to about 10 times, and the obtained extract may be filtered, concentrated under reduced pressure, and dried, thereby obtaining a Veronicastrum sibiricum L. Pennell crude extract.

Also, a non-polar soluble extract fraction soluble in a non-polar solvent such as n-hexane, methylene chloride, ethyl acetate, and butanol and a polar soluble extract fraction soluble in a polar solvent such as butanol and water may be obtained by adding water having a volume (v/w%) that is about 0.0005 to about 500 folds, preferably, about 0.05 to about 50 folds the weight of the crude extract obtained above, preferably, about 60% to about 90% of the ethanol crude extract weight and performing a general fraction process by using n-hexane, methylene chloride, ethyl acetate, and butanol.

Also, general fraction processes that are well known in the field may be additionally performed (Harborne J.B. Phytochemical methods: A guide to modern techniques of plant analysis. 3rd Ed. pp 6-7, 1998).

The present inventors has identified that the composition is useful as a pharmaceutical composition or health functional food for prevention and treatment of inflammation, allergies, and asthma due to the confirmation, through various animal experiments, that a Veronicastrum sibiricum L. Pennell (genus Veronicastrum) extract exhibits better anti-inflammatory, anti-allergic and asthma-inhibitory activity than a Veronica longifolia (genus *Pseudolysimachion)* extract obtained via an existing method, the animal experiments conducted using the Veronicastrum sibiricum L. Pennell (genus Veronicastrum) extract instead of the Veronica longifolia (genus *Pseudolysimachion)* extract disclosed in KR10-2006-0125489 and including: evaluation of inhibition of leukotriene production (Experimental Example 1); effects on the total number of cells in bronchoalveolar lavage (BAL) fluid by using male Balb/c mice (Experimental Example 2); effects on the number of neutrophil cells relative to the total number of cells in the BAL fluid (Experimental Example 3); effects on the number of Neutrophil+/Gr-1+ absolute cells in the BAL fluid (Experimental Example 4); effects on the number of CD11b+/Gr-1+ absolute cells from among lung cells (Experimental Example 5); effects on the number of CD4+/CD3+ absolute cells from among lung cells (Experimental Example 6); effects on the number of Macrophage+/CD1 1b+ absolute cells from among lung cells (Experimental Example 7); effects on the expression of inflammation factors in the BAL fluid (Experimental Example 8); and the like.

Therefore, the present disclosure provides a pharmaceutical composition or a health functional food including the Veronicastrum sibiricum L. Pennell extract, which is obtained according to the above preparation method, as an active ingredient for the prevention and treatment of allergies, and asthma.

Also, Veronicastrum sibiricum L. Pennell is a medicine that has been eaten or used as a herb medicine, and the Veronicastrum sibiricum L. Pennell extract has no toxicity or side effects.

The term "prevention" used herein indicates all activities for inhibiting or delaying inflammation, allergies, or asthma by administering a composition including the above extract. Also, the term "treatment" used herein includes all activities for improving or advantageously changing symptoms of diseases by administering the composition including the extract.

According to another aspect, the present disclosure provides a treatment method for treating allergies, or asthma, the treatment method including administration of an extract of Veronicastrum sibiricum L. Pennell to patients with allergies, or asthma.

According to another aspect, the present disclosure provides a use of the Veronicastrum sibiricum L. Pennell extract for preparation of medicament for treating patients suffering from allergies, and/or asthma.

A pharmaceutical composition including a purified extract may be formulated in oral dosage form such as powder, granules, tablets, capsules, suspension, emulsion, syrup, or aerosol, topical preparation, and a sterilized injection solution, according to each existing method. Carriers, excipients, and diluents that may be included in a composition including the extract may be, for example, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. The formulation may be performed using generally-used excipients and diluents such as fillers, weighting agents, binders, wetting agents, disintegrating agents, and surfactants. The solid preparation for oral administration may include tablets, pills, powder, granules, capsules, and the like, and such solid preparation may be prepared by adding at least one excipient such as starch, calcium carbonate, sucrose, lactose, or gelatin to the above extract and fraction. Also, in addition to a simple excipient, lubricants such as magnesium stearate and talc may be used. Liquid preparation for oral administration may include suspension, emulsion, syrup, and the like, and in addition to commonly-used diluents such as water and liquid paraffin, various excipients such as wetting agents, flavorings, odorants, and preservatives may be included Pharmaceutical preparation for parenteral administration includes sterilized aqueous solution, a non-aqueous solvent, suspension, emulsion, lyophilized preparation, and suppository.

Examples of the suspension and the non-aqueous solvent may include propylene glycol, polyethylene glycol, vegetable oils such as olive oil, injectable ester such as ethyl oleate, and the like. Base of suppository may include witepsol, macrogol, tween 61, cacao butter, laurin, glycerogelatin, and the like.

A desirable dosage of the pharmaceutical composition including the extract of the present disclosure may vary depending on conditions and weights of patients, severity of diseases, medicine types, administration routes and periods, but may be appropriately selected by one of ordinary skill in the art. However, to obtain desirable effects, it is recommended to administer the pharmaceutical composition including the extract of the present disclosure in a range from about 0.0001 to about 100 mg/kg, preferably, from about 0.001 to about 100 mg/kg a day. The pharmaceutical composition may be administered once a day or several times a day. The dosage does not limit the scope of the present disclosure in any aspect.

The extract may be administered to mammals such as rats, mice, domestic animals, and human in various routes. All administration methods may be contemplated. For example, the administration may be made orally, rectally or by intravenous, intramuscular, subcutaneous, intrauterine dural, and intracere broventricular injection.

The pharmaceutical composition may include about 0.1 to about 50 wt% of the extract relative to the total weight of the composition.

The pharmaceutical preparation for parenteral administration includes sterilized aqueous solution, a non-aqueous solvent, suspension, emulsion, lyophilized preparation, and suppository. Examples of the suspension and the non-aqueous solvent may include propylene glycol, polyethylene glycol, vegetable oils such as olive oil, injectable ester such as ethyl oleate, and the like. Base of suppository may include witepsol, macrogol, tween 61, cacao butter, laurin, glycerogelatin, and the like.

A desirable dosage of the extract including the extract of the present disclosure may vary depending on conditions and weights of patients, severity of diseases, medicine types, administration routes and periods, but may be appropriately selected by one of ordinary skill in the art. However, to obtain desirable effects, it is recommended to administer the extract of the present disclosure in a range from about 0.0001 to about 100 mg/kg, preferably, from about 0.001 to about 100 mg/kg, once a day or several times a day. The content of extract in the composition may range from about 0.0001 to about 50 wt% of the total weight of the composition.

The pharmaceutical composition may be administered to mammals such as rats, mice, domestic animals, and human in various routes. All administration methods may be contemplated. For example, the administration may be made orally, rectally or by intravenous, intramuscular, subcutaneous, intrauterine dural, and intracere broventricular injection.

Also, the present disclosure provides a treatment method for treating patients with allergies, and asthma, the treatment method including the administration of the Veronicastrum sibiricum L. Pennell (genus Veronicastrum) extract to the patients with allergies, and asthma.

Also, the present disclosure provides a use of the Veronicastrum sibiricum L. Pennell (genus Veronicastrum) extract for preparation of medicament for treating patients with allergies, and asthma.

Also, the present disclosure provides a health functional food including the Veronicastrum sibiricum L. Pennell (genus Veronicastrum) extract as an active ingredient for prevention and improvement of allergies, and asthma

The term "health functional food" defined herein indicates food manufactured and processed by using base materials or ingredients having functionality useful to humans, according to the Law for Health Functional Foods 6727 in South Korea. The term "functionality" indicates ingestion to adjust nutrients with regard to a structure and functions of a human body or obtain effects advantageous to health care such as physiological effects.

The health functional food for preventing or improving allergies, and asthma includes about 0.01 to about 95%, preferably, about 1 to about 80% of the extract relative to the total weight of the composition.

Also, for the purpose of preventing or improving allergies, and asthma, the health functional food may be manufactured and processed in the form of pharmaceutically acceptable administration such as powder, granules, tablets, capsules, pills, suspension, emulsion, or syrup or the form of tea bag, leached tea, health beverage, or the like.

Also, the present disclosure provides a health functional food including the Veronicastrum sibiricum L. Pennell (genus Veronicastrum) extract as an active ingredient for the prevention and improvement of allergies, and asthma

Also, the present disclosure provides food or a food additive including the extract of Veronicastrum sibiricum L. Pennell (genus Veronicastrum) as an active ingredient for the prevention and improvement of allergies, and asthma

Also, the health functional food may additionally include a food additive, and the suitability as "food additives" is determined according to standards and criteria of items in accordance with the general provisions and general analytical method of the Korean Food Additive Code approved by the Ministry of Food and Drug Safety, unless otherwise specified.

Examples of products listed in the "Korean Food Additive Code" may include ketones, chemical products such as glycine, potassium citrate, nicotinic acid and cinnamic acid, natural additives such as a persimmon color, licorice extract, crystalline cellulose and guar gum, and mixed formulations such as monosodium L-glutamate, alkali agents for noodles, preservative formulation and tar color formulation.

Examples of the functional food including the extract may include confectionary such as bread, rice cake, dried fruit, candy, chocolate, chewing gum, and jam, ice cream products such as ice cream, frozen dessert, and ice cream powder, dairy products such as milk, low-fat milk, lactose-free milk, processed milk, goad milk, fermented milk, buttermilk, condensed milk, milk cream, butter oil, butter oil, natural cheese, processed cheese, milk powder, and whey, meat products such as processed meat products, egg products, and hamburger, fish and meat products including processed fish and meat products such as fish cake, ham, sausage, and bacon, noodles such as instant noodle, dried noodle, raw noodle, instant fried noodle, instant non-fried noodle, processed noodle, frozen noodle, pasta, fruit drink, vegetable drink, carbonated drink, soy milk, lactic acid beverage such as yogurt, beverage such as mixed drink, seasonings such as soy sauce, soybean paste, red pepper paste, chunjang, cheonggukjang, mixed soy paste, vinegar, sauces, tomato ketchup, curry, and dressing, margarine, shortening, and pizza. However, one or more embodiments are not limited thereto.

The health functional beverage composition has no particular limitation on components other than the inclusion of the above extract as an essential ingredient at an indicated ratio, and may additionally include flavorings or natural carbohydrates like existing beverages. Examples of the above natural carbohydrates include general sugar such as monosaccharides (e.g., glucose, fructose, etc.), disaccharides (e.g., maltose, sucrose, etc.), and polysaccharides (e.g., dextrin, cyclodextrin, etc.), and sugar alcohols such as xylitol, sorbitol, and erythritol. In addition to those described above, natural flavorings (thaumatin, stevia extracts (e.g., rebaudioside A, glycyrrhizin, etc.)), and synthetic flavoring agents (e.g., saccharin, aspartame, etc.) may be advantageously used as flavorings. A ratio of the natural carbohydrates may generally range from about 1 to about 20 g, preferably, from about 5 to about 12g, per 100 mℓ of the composition.

In addition to those described above, the composition may contain various nutrients, a vitamin, a mineral (an electrolyte), flavorings such as synthetic and natural flavorings, a coloring agent and an improving agent (cheese, chocolate, etc.), pectic acid and the salt thereof, alginic acid and the salt thereof, organic acid, a protective colloidal adhesive, a pH regulator, a stabilizer, a preservative, glycerin, alcohol, a carbonizing agent used in a carbonate beverage, and the like. The other components than the aforementioned components may be fruit pulp for preparing natural fruit juice, a fruit juice beverage, and vegetable juice. Such components may be used independently or in combination. A ratio of the additives is not so important, but is generally selected in a range from about 0 to about 20 parts by weight per 100 parts by weight.

Also, the extract of the present disclosure may be added to food or beverages for prevention of purposed diseases. In this case, the amount of the extract in food or beverages may range from about 0.01 wt% to about 15 wt% of the total food weight, and a health beverage composition may be added at a ratio of between about 0.02 g to about 5 g, preferably between about 0.3 g and 1 g per 100 mℓ.

While the health functional food is prepared, the present extract added to food including beverages may be appropriately adjusted according to necessity.

### ADVANTAGEOUS EFFECTS OF DISCLOSURE

It has been ascertained, through various animal experiments, that a Veronicastrum sibiricum L. Pennell extract (genus Veronicastrum) according to the present disclosure exhibits excellent anti-inflammatory, anti-allergic, and asthma-inhibitory activity, the animal experiments including: an evaluation of inhibition of leukotriene production (Experimental Example 1); effects on the total number of cells in bronchoalveolar lavage (BAL) fluid by using male Balb/c mice (Experimental Example 2); effects on the number of neutrophil cells relative to the total number of cells in the BAL fluid (Experimental Example 3); effects on the number of Neutrophil+/Gr-1+ absolute cells in BAL fluid (Experimental Example 4); effects on the number of CD11b+/Gr-1+ absolute cells from among lung cells (Experimental Example 5); effects on the number of CD4+/CD3+ absolute cells from among lung cells (Experimental Example 6); effects on the number of Macrophage+/CD11b+ absolute cells from among lung cells (Experimental Example 7); effects on the expression of inflammation factors in BAL fluid (Experimental Example 8); and the like.

### BEST MODE

It would be obvious to one of ordinary skill in the art that various modifications and variations may be made for compositions, use, and preparation of the present disclosure without departing from the spirit or scope of the present disclosure.

One or more embodiments of the present disclosure are described in detail, but it should be understood that the present disclosure is not limited to those embodiments in any manner.

However, embodiments and experimental examples below are merely examples without limiting the scope of the present disclosure, and the present disclosure is not limited to those embodiments and experimental examples.

### Comparative example 1: preparation of a crude extract of Veronica Longifolia

As disclosed in the related art (KR 10-2006-0125489), the crude extract of Veronica Longifolia was prepared as follows.

1.1 kg of dried and pulverized Veronica longifolia (Pseudolysimachion) (genus Veronica) was added to 5 L of methanol and agitated at a room temperature for 24 hours, thus collecting a supernatant through vacuum filtration. After the above process was repeated twice to collect the supernatant, the supernatant was concentrated by a reduced pressure concentrator (EYELA, N-2100, JAPAN) under reduced pressure to collect 100.5 g of the methanol crude extract of Veronica longifolia (hereinafter, referred to as "VLM") which was used as a comparative sample in the following Experimental Example.

### Example 1: preparation of a crude extract of Veronicastrum sibiricum L. Pennell

### 1-1. Preparation of a methanol extract of Veronicastrum sibiricum L. Pennell

1.16 kg of dried and pulverized Veronicastrum sibiricum L. Pennell (genus Veronicastrum) (cultivated in Ansan, Gyeonggi-do, South Korea) was added to methanol. 4L of a mixture each was exposed to ultrasonic waves by using an ultrasonic extractor (5510R-DTH, Bransonic) at a temperature of about 60 °C for two hours, and the exposure was repeated three times to prepare the methanol extract of Veronicastrum sibiricum L. Pennell. Then, the extract was completely dried through reduced pressure concentration using the reduced pressure concentrator (EYELA, N-2100, JAPAN) and freeze drying using a freeze dryer (FDU-2100, Lab corporation) at a temperature of about 40 °C, and 120.68 g of the methanol extract of dried Veronicastrum sibiricum L. Pennell (hereinafter, referred to as "VSM") was obtained and stored at a temperature of about - 20 °C.

### 1-2. Preparation of a water extract of Veronicastrum sibiricum L. Pennell

1.08 kg of dried and pulverized Veronicastrum sibiricum L. Pennell (genus Veronicastrum) (cultivated in Ansan, Gyeonggi-do, South Korea) was added to distilled water. 4L of a mixture each was exposed to ultrasonic waves by using the ultrasonic extractor (5510R-DTH, Bransonic) at a temperature of about 80 °C for two hours, and the exposure was repeated three times to prepare the water extract of Veronicastrum sibiricum L. Pennell. Then, the extract was completely dried through reduced pressure concentration using the reduced pressure concentrator (EYELA, N-2100, JAPAN) and freeze drying using the freeze dryer (FDU-2100, Lab corporation) at a temperature of about 40 °C, and 52.3 g of the water extract of dried Veronicastrum sibiricum L. Pennell (hereinafter, referred to as "VSW") was obtained and stored at a temperature of about - 20 °C.

### 1-3. Preparation of a 25% ethanol extract of Veronicastrum sibiricum L. Pennell

10.0 g of dried and pulverized Veronicastrum sibiricum L. Pennell (genus Veronicastrum) (cultivated in Ansan, Gyeonggi-do, South Korea) was added to a 25% ethanol aqueous solution. 4L of a mixture each was exposed to ultrasonic waves by using an ultrasonic extractor (5510R-DTH, Bransonic) at a temperature of about 80 °C for two hours, and the exposure was repeated three times to prepare the 25% ethanol extract of Veronicastrum sibiricum L. Pennell. Then, the extract was completely dried through reduced pressure concentration using the reduced pressure concentrator (EYELA, N-2100, JAPAN) and freeze drying using the freeze dryer (FDU-2100, Lab corporation) at a temperature of about 40 °C, and 1.6 g of the 25% ethanol extract of dried Veronicastrum sibiricum L. Pennell (hereinafter, referred to as "VS25M") was obtained and stored at a temperature of about -20 °C.

### 1-4. Preparation of a 50% ethanol extract of Veronicastrum sibiricum L. Pennell

10.0 g of dried and pulverized Veronicastrum sibiricum L. Pennell (genus Veronicastrum) (cultivated in Ansan, Gyeonggi-do, South Korea) was added to a 50% ethanol aqueous solution. 4L of a mixture each was exposed to ultrasonic waves by using an ultrasonic extractor (5510R-DTH, Bransonic) at a temperature of about 80 °C for two hours, and the exposure was repeated three times to prepare the 50% ethanol extract of Veronicastrum sibiricum L. Pennell. Then, the extract was completely dried through reduced pressure concentration using the reduced pressure concentrator (EYELA, N-2100, JAPAN) and freeze drying using the freeze dryer (FDU-2100, Lab corporation) at a temperature of about 40 °C, and 1.7 g of the 50% ethanol extract of dried Veronicastrum sibiricum L. Pennell (hereinafter, referred to as "VS50E") was obtained and stored at a temperature of about -20 °C.

### 1-5. Preparation of a 75% ethanol extract of Veronicastrum sibiricum L. Pennell

10.0 g of dried and pulverized Veronicastrum sibiricum L. Pennell (genus Veronicastrum) (cultivated in Ansan, Gyeonggi-do, South Korea) was added to a 75% ethanol aqueous solution. 4L of a mixture each was exposed to ultrasonic waves by using an ultrasonic extractor (5510R-DTH, Bransonic) at a temperature of about 80 °C for two hours, and the exposure was repeated three times to prepare the 75% ethanol extract of Veronicastrum sibiricum L. Pennell. Then, the extract was completely dried through reduced pressure concentration using the reduced pressure concentrator (EYELA, N-2100, JAPAN) and freeze drying using the freeze dryer (FDU-2100, Lab corporation) at a temperature of about 40 °C, and 12.9 g of the 75% ethanol extract of dried Veronicastrum sibiricum L. Pennell (hereinafter, referred to as "VS75E") was obtained and stored at a temperature of about -20 °C.

### 1-6. Preparation of an ethanol extract of Veronicastrum sibiricum L. Pennell

10.0 g of dried and pulverized Veronicastrum sibiricum L. Pennell (genus Veronicastrum) (cultivated in Ansan, Gyeonggi-do, South Korea) was added to 100% ethanol. 4L of a mixture each was exposed to ultrasonic waves by using an ultrasonic extractor (5510R-DTH, Bransonic) at a temperature of about 80 °C for two hours, and the exposure was repeated three times to prepare the ethanol extract of Veronicastrum sibiricum L. Pennell. Then, the extract was completely dried through reduced pressure concentration using the reduced pressure concentrator (EYELA, N-2100, JAPAN) and freeze drying using the freeze dryer (FDU-2100, Lab corporation) at a temperature of about 40 °C, and 0.5 g of the 100% ethanol extract of dried Veronicastrum sibiricum L. Pennell (hereinafter, referred to as "VSE") was obtained and stored at a temperature of about -20 °C.

### Example 2: preparation of a non-polar soluble extract of Veronicastrum sibiricum L. Pennell

120.68 g of the methanol extract of Veronicastrum sibiricum L. Pennell of Example 1 was dissolved in water. Then, a generated solution was fractioned to 4L of hexane to obtain an aqueous solution layer. Then, the solution was fractioned again to 4L of ethyl acetate to collect an ethyl acetate solution layer, thus preparing an ethyl acetate extract of Veronicastrum sibiricum L. Pennell. Then, the extract was completely dried through reduced pressure concentration using the reduced pressure concentrator (EYELA, N-2100, JAPAN) and freeze drying using the freeze dryer (FDU-2100, Lab corporation) at a temperature of about 40 °C, and 12.6 g of an ethyl acetate soluble fraction of dried Veronicastrum sibiricum L. Pennell (hereinafter, referred to as "VSEA") was obtained and stored at a temperature of about -20 °C.

### Experimental Example 1: Evaluation of inhibition of leukotriene production

In order to confirm the inhibition of leukotriene production of the sample in Preparation Example, the following experiment was conducted by applying the method stated in the literature (Goulet, J. L., et al., J Immunol, 164(9), 4899-4907 (2000).

### 1-1. Experiment method

In order to confirm the inhibitory activity of 5-lipoxygenase of samples of the above examples, leukotrienes were quantitated by using a method disclosed in the literature (Goulet, J. L., et al., J Immunol, 164(9), 4899-4907 (2000), and the result is shown in Table 2. In Table 2, montelukast (PHR1603, Sigma-Aldrich), the positive control group, had the final concentration of about 100 µM in the medium, and other test agents in Preparation Examples 1 and 2 each had the final concentration of about 10 µg/ml to about 50 µg/ml, and test agents in Preparation Examples 3 to 7 each had the final concentration of about 100 µg/ml to about 200 µg/ml.

RBL-2H3 cells (22256, KCLB), dispersed to 5×10⁵, were treated by adjusting the final concentration of the extract to range from about 10 µg/ml to about 50 µg/ml or from about 100 µg/ml to about 200 µg/ml. After 10 minutes of the sample treatment, leukotrienes were induced by treating 20 µg of calcium ionophore (A23187, Sigma-Aldrich). After 10 minutes, the supernatant was collected, and leukotrienes were quantified at 405 nm by an enzyme-linked immunosorbent assay (ELISA) reader (Powerwave XS, Biotek) according to an ELISA method (ADI-900-070, Ezno lifescience)

### 1-2. Experiment result

Results of measuring the production amount of leukotrienes on the samples were shown in Tables 1 and 2. According to Examples 1 and 2, samples prepared in Examples 1 and 2 had no inhibiting effects at a concentration of 10 µg/ml. However, at a centration of 50 µg/ml, the sample in Example 2 showed more effects on the inhibition of the leukotriene production than the sample in Example 1, and thus, it was confirmed that the sample in Example 2 effectively inhibits respiratory inflammation (Table 1). At a concentration of 100µg/ml, the sample prepared in Example 1 had effects on the inhibition of the leukotriene production except for Examples 1-2 to 1-4, and in particular, the sample in Example 1-6 showed a potent inhibiting effect. At a concentration of 200µg/ml, the samples prepared in Examples 1-3 to 1-6, except the sample in Example 1-2, were effective in inhibiting the respiratory inflammation, and especially, the sample in Example 1-6 was more effective in the inhibition of respiratory inflammation than other examples (Table 2).

**[Table 1]**

| Experiment result of ability to inhibit leukotriene production | | | |
|---|---|---|---|
| Classification | | Leukotriene concentration (pg/ml) | Inhibition rate (based on induction group) |
| Normal control group | | 136.5±7.9 | |
| Induction group | | 2603.5±400.9 | |
| Positive control group | 100 µM | 684.4±26.8 | 74% |
| Example 1-1 | 10 µg/ml | 2794.6±627 | - |
| | 30 µg/ml | 2279.1±771.8 | 12% |
| | 50 µg/ml | 2557.3±280.7 | 2% |
| Example 2 | 10 µg/ml | 2689.9±115.4 | - |
| | 30 µg/ml | 2503.9±293.8 | 4% |
| | 50 µg/ml | 216.81288.7± | 51% |

**[Table 2]**

| Experiment result of ability to inhibit leukotriene production | | | |
|---|---|---|---|
| Classification | | Leukotriene concentration (pg/ml) | Inhibition rate (based on induction group) |
| Normal control group | | 161.7±3.0 | |
| Induction group | | 2656.8±30.1 | |
| Positive control group | 100 µM | 167.9±0.5 | 94% |
| Example 1-2 | 100 µg/ml | 2557.7±285.3 | 4% |
| | Example 1-3 | 2723.8±466.3 | - |
| | Example 1-4 | 2645.1±369.4 | - |
| | Example 1-5 | 2125.9±112.0 | 20% |
| | Example 1-6 | 14930±393.0 | 44% |
| Example 1-2 | 200 µg/ml | 2910.9±467.8 | - |
| | Example 1-3 | 1797.5±336.6 | 32% |
| | Example 1-4 | 1550.8±192.2 | 42% |
| | Example 1-5 | 764.5±55.5 | 70% |
| | Example 1-6 | 708.1±83.4 | 73% |

### Experimental example 2: measuring the total number of cells in BAL fluid

In order to confirm the effects of the samples in above Examples on the total number of cells in BAL fluid, the following experiment was conducted by applying the method disclosed in the literature (Schins et al., Toxicol Appl Pharmacol. 195(1), 1-11 (2004) and Smith et al., Toxicol Sci, 93(2), 390-399 (2006)).

### 2-1. Experiment method

Six male Balb/C mice were grouped, and 0.25mg/ml coal, 10mg/ml fly ash, and 0.2 mg/ml diesel exhaust particles (DEP), which are components of fine dust, were mixed to have an 8% final Alum concentration, and 50µl of the mixture of fine dust was directly inoculated to the airway and nose of subject animals on 3^{rd} and 6^{th} days of the experiment except for the normal control group, according to the Intra-Nazal-Trachea (INT) injection method disclosed in the literature (Lim et al., Free Radic Biol Med. 25(6), 635-644. (1998)) The positive control group (N-acetylcysteine, Sigma A7250), and the samples in Examples were diluted with a 0.5% sodium carboxymethyl cellulose (CMC, 419273, Sigma-Aldrich) solution at a concentration of 200mg/kg and orally administered to the subject animals of each group every day (10 days). On the 11^{th} day after the experiment, an autopsy was performed on the mice, and the BAL fluid was collected.

### 2-2 Experiment result

Table 3 shows a result of measuring effects of the samples on the total number of cells in the BAL fluid. It was confirmed that all of the samples were effective in reducing the inflammatory level because the total number of BAL cells was reduced compared to the induction group. Moreover, the total number of BAL cells in Example 1-1 was smaller than that in Comparative Example 1, and the total number of BAL cells in the treatment group of Example 2 was smaller than the total number of BAL cells in Example 1-1, and thus, it was found that the sample in Example 2 had excellent inflammation inhibitory activity.

**[Table 3]**

| Effects on the total number of cells in BAL fluid | | |
|---|---|---|
| Classification | Total BAL cell (×10⁵ cells/ml) | Inhibition rate (based on induction group) |
| Normal control group | 11.3±4.48 | |
| Induction group | 231.0±30.48 | |
| Positive control group | 117.3±21.35 | 49% |
| Example 1-1 | 147.5±30.38 | 36% |
| Example 2 | 121.3±19.47 | 47% |
| Comparative Example 1 | 206.5±18.71 | 11% |

### Experimental Example 3: measuring a ratio of the number of neutrophil cells to the total number of cells in BAL fluid

In order to confirm the effects of the samples in above Examples on a ratio of the number of neutrophil cells to the total number of cells in BAL fluid, the following experiment was conducted by applying the method disclosed in the literature (Schins et al., Toxicol Appl Pharmacol. 195(1), 1-11 (2004) and Smith et al., Toxicol Sci, 93(2), 390-399 (2006)).

### 3-1. Experiment method

The experiment was conducted according to the same method as that in Experimental Example 2. In the collected BAL fluid, neutrophils were stained according to a Diff-Qick staining method (Takano et al., Am J Respir Crit Care Med, 156(1), 36-42. (1997), Hemacolor Rapid staining of blood smear, 1.11661.0001, Merck) and observed.

### 3-2 Experiment result

Table 4 shows results of measuring the effects of the samples on the ratio of the number of neutrophil cells to the total number of cells in the BAL fluid. It was confirmed that all of the samples effectively decreased the inflammatory levels because the ratio of the number of neutrophil cells was reduced compared to the induction group. Moreover, the ratio of the number of neutrophil cells to the total number of cells in the BAL fluid was lower in Example 1-1 than in Comparative Example 1, and the ratio was lower in Example 2 than in Example 1-1. Thus, it was found that the sample in Example 2 had excellent inflammation inhibitory activity.(Table 4)

**[Table 4]**

| Effects on the ratio of neutrophil cells to the total number of cells in BAL fluid | | |
|---|---|---|
| Classification | Cell percentage (%) | Inhibition rate (based on induction group) |
| Normal control group | 5.3±1.0 | |
| induction group | 99.0±9.0 | |
| Positive control group | 27.5±7.9 | 72% |
| Example 1-1 | 64.5±6.3 | 36% |
| Example 2 | 39.0±6.4 | 61% |
| Comparative example 1 | 92.2±5.7 | 9% |

### Experimental Example 4: measuring the number of Neutrophil+/Gr-1+ absolute cells in BAL fluid

In order to confirm the effects of the samples in above Examples on the number of Neutrophil+/Gr-1+ absolute cells in BAL fluid, the following experiment was conducted by applying the method disclosed in the literature (Beutner EH., Bacteriological Reviews., 25(1):49-76, ((1961)).

### 4-1. Experiment method

The experiment was conducted according to the same method as that in Experimental Example 2 except for the measurement of the number of cells in the BAL fluid. A specific fluorescence fluorescent antibody staining method was performed on the collected BAL fluid by using fluorescence-labeled Gr-1 antibodies (553128, BD Biosciences, San Jose, CA, USA), and the number of Neutrophil+/Gr-1+ absolute cells in the total leukocytes was measured according to a Fluorescence-activated cell sorting (FACS) (BD Biosciences, San Jose, CA, USA) method.

### 4-2. Experiment result

Table 5 shows the result of measuring the number of Neutrophil+/Gr-1+ absolute cells in the BAL fluid in the samples. It was confirmed that the number of Neutrophil+/Gr-1+ absolute cells in each sample is reduced compared to the induction group. Moreover, the number of Neutrophil+/Gr-1+ absolute cells in the treatment group of Example 2 was less than that in Example 1-1, and thus, it was found that the sample in Example 2 had excellent inflammation inhibitory activity. (Table 5)

**[Table 5]**

| Effects on the number of Neutrophil+/Gr-1+ absolute cells in BAL fluid | | |
|---|---|---|
| Classification | Cell number (×10⁴ cells/ml) | Inhibition rate (based on induction group) |
| Normal control group | 1.6±0.7 | |
| induction group | 122.0±25.5 | |
| Positive control group | 86.9±16.9 | 29% |
| Example 1-1 | 62.8±8.5 | 49% |
| Example 2 | 67.8±14.2 | 44% |

### Experimental Example 5: measuring the number of CD11b+/Gr-1+ absolute cells from among lung cells

In order to confirm the effects of the samples in above Examples on the number of CD11b+/Gr-1+ absolute cells from among the lung cells, the following experiment was conducted by applying the method disclosed in the literature (Beutner EH., Bacteriological Reviews., 25(1):49-76, ((1961)).

### 5-1. Experiment method

The experiment was conducted according to the same method as that in Experimental Example 2 except for the measurement of the number of cells in the BAL fluid. The specific fluorescence fluorescent antibody staining method was performed on the collected lung by using fluorescence-labeled CD11b antibodies (553310, BD Biosciences, San Jose, CA, USA) and Gr-1 antibodies (553128, BD Biosciences, San Jose, CA, USA), and the number of CD11b+/Gr-1+ absolute cells in the total number of lung cells was measured according to the FACS (BD Biosciences, San Jose, CA, USA) method.

### 5-2. Experiment result

Table 6 shows the result of measuring the number of CD11b+/Gr-1+ absolute cells from among the lung cells in the samples. It was confirmed that the number of CD11b+/Gr-1+ absolute cells in each sample was reduced compared to that in the induction group, and moreover, the number of CD11b+/Gr-1+ absolute cells in Example 2 was less than that in Example 1-1. Thus, it was found that the sample in Example 2 had excellent inflammation inhibitory activity.

**[Table 6]**

| Effects on the number of CD11b+/Gr-1+ absolute cells from among the lung cells | | |
|---|---|---|
| Classification | Cell number (×10⁵ cells/ml) | Inhibition rate (based on induction group) |
| Normal control group | 34.5±3.35 | |
| induction group | 81.5±0.96 | |
| Positive control group | 27.6±8.95 | 66% |
| Example 1-1 | 71.8±0.39 | 12% |
| Example 2 | 37.7±8.82 | 54% |

### Experimental Example 6: measuring the number of CD4+/CD3+ absolute cells from among lung cells

In order to confirm the effects of the samples in above Examples on the number of CD4+/CD3+ absolute cells, the following experiment was conducted by applying the method disclosed in the literature (Beutner EH., Bacteriological Reviews., 25(1):49-76, ((1961)).

### 6-1. Experiment method

The experiment was conducted according to the same method as that in Experimental Example 2 except for the measurement of the number of cells in the BAL fluid. The specific fluorescence fluorescent antibody staining method was performed on the collected lung by using fluorescence-labeled CD4 antibodies (550280, BD Biosciences, San Jose, CA, USA) and Gr-1 antibodies (554829, BD Biosciences, San Jose, CA, USA), and the number of CD4+/CD3+ absolute cells in the total number of lung cells was measured according to the FACS (BD Biosciences, San Jose, CA, USA) method.

### 6-2. Experiment result

Table 7 shows the result of measuring the number of CD4+/CD3+ absolute cells from among the lung cells in the samples. It was confirmed that the number of CD4+/CD3+ absolute cells in each sample was reduced compared to that in the induction group, and moreover, the number of CD4+/CD3+ absolute cells in Example 2 was less than that in Example 1-1. Thus, it was found that the sample in Example 2 had excellent inflammation inhibitory activity.

**[Table 7]**

| Effects on the number of CD4+/CD3+ absolute cells from among the lung cells | | |
|---|---|---|
| Classification | Cell number (×10⁵ cells/ml) | Inhibition rate (based on induction group) |
| Normal control group | 75.6±3.14 | |
| induction group | 132.6±7.21 | |
| Positive control group | 81.4±0.13 | 39% |
| Example 1-1 | 103.6±5.26 | 22% |
| Example 2 | 80.2±0.34 | 40% |

### Experimental Example 7: measuring the number of Macrophage+/CD11b+ absolute cells from among lung cells

In order to confirm the effects of the samples in above Examples on the number of Macrophage+/CD11b+ absolute cells, the following experiment was conducted by applying the method disclosed in the literature (Beutner EH., Bacteriological Reviews., 25(1):49-76, ((1961)).

### 7-1. Experiment method

The experiment was conducted according to the same method as that in Experimental Example 2 except for the measurement of the number of cells in the BAL fluid. The specific fluorescence fluorescent antibody staining method was performed on the collected lung by using fluorescence-labeled CD11b antibodies (553310, BD Biosciences, San Jose, CA, USA), and the number of Macrophage+/CD11b+ absolute cells in the total number of lung cells was measured according to the FACS (BD Biosciences, San Jose, CA, USA) method.

### 7-2. Experiment result

Table 8 shows the result of measuring the number of Macrophage+/CD11b+ absolute cells from among the lung cells in the samples. It was confirmed that the number of Macrophage+/CD11b+ absolute cells in each sample was reduced compared to that in the induction group, and moreover, the number of Macrophage+/CD11b+ absolute cells in Example 2 was less than that in Example 1-1. Thus, it was found that the sample in Example 2 had excellent inflammation inhibitory activity.

**[Table 8]**

| Effects on the number of Macrophage+/CD11b+ absolute cells from among lung cells | | |
|---|---|---|
| Classification | Cell number (×10⁵ cells/ml) | Inhibition rate (based on induction group) |
| Normal control group | 35.2±3.04 | |
| induction group | 88.8±0.22 | |
| Positive control group | 35.4±8.10 | 60% |
| Example 1-1 | 79.2±0.74 | 11% |
| Example 2 | 45.6±9.96 | 49% |

### Experimental Example 8: determination on the expression of inflammation factors in the BAL fluid

In order to confirm the effects of the samples in above Examples on the expression of inflammation factors in the BAL fluid, the following experiment was conducted by applying the method disclosed in the literature (Brandt EB et al., J. Allergy Clin. Immunol., 132(5):1194-1204, (2013)).

A determination test using the ELISA was performed to determine the expression of inflammation factors such as IL-17A, TNF-α MIP2, and CXCL-1 in the BAL fluid

### 8-1. Experiment procedure

The experiment was conducted according to the same method as that in Experimental Example 2 except for the measurement of the number of cells in the BAL fluid. Levels of IL-17A, TNF-α MIP2, and CXCL-1 were determined using the ELISA. IL-17A antibodies (M1700, R&D Systems, Minneapolis, USA), TNF-α antibodies (MTA00B, R&D Systems, Minneapolis, USA), MIP2 antibodies (MM200, R&D Systems, Minneapolis, USA), and CXCL-1 antibodies (MKC00B, R&D Systems, Minneapolis, USA) were diluted with a buffer solution and coated micro-wells and then incubated at a temperature of 4 °C for 16 hours. Each well was washed with the buffer solution three times, and a 10-fold diluted serum was dispensed at 100 µl per well.

After being left at a room temperature for one hour, the well was washed twice, and 100 µl of Avidin-HRP-combined antibodies (DY007, R&D Systems, Minneapolis, USA) was treated. Then, the well was left at the room temperature for one hour and washed again. A TMB substrate (DY007, R&D Systems, Minneapolis, USA) was dispensed at 100 µl per well, and the well was left in shadow for 30 minutes. Then, the well is treated with 50 µl of a stop solution (DY007, R&D Systems, Minneapolis, USA), and the absorbance of the solution was determined at 450 nm.

### 8-2. Experiment result

As shown in Table 9, the inflammatory factors (IL-17A, TNF-α, MIP2, and CXCL-1) in groups treated with the samples were reduced more than those in the induction group. Compared to Example 1-1, the group treated with the sample in Example 2 showed lower expression of IL-17A, TNF-α, MIP2, and CXCL-1, and thus, it was found that the sample in Example 2 had excellent respiratory inflammation inhibitory activity.

**[Table 9]**

| Effects on expression of inflammatory factors in BAL fluid | | | | |
|---|---|---|---|---|
| Classifica tion | Concentration (pg/ml)/inhibition rate (%) based on induction group | | | |
| | IL-17A | TNF-α | MIP2 | CXCL-1 |
| Normal control group | 10.45±3.16 | 87.53±12.73 | 79.01±10.38 | 98.73±11.53 |
| induction group | 25.11±5.62 | 166.00±34.77 | 136.13±9.19 | 605.36±87.17 |
| Positive control group | 19.48±1.89/22% | 124.57±5.74/25% | 118.75±9.26/13% | 229.27±23.21/62% |
| Example 1-1 | 12.53±2.83/50% | 78.16±9.48/53% | 121.46±19.77/11% | 463.24±105.91/23 % |
| Example 2 | 9.94±2.33/60% | 72.43±15.35/56% | 102.92±9.10/24% | 373.88±62.31/38% |

### MODE OF DISCLOSURE

Hereinafter, a formulation method and kinds of carriers will be described, but the present disclosure is not limited thereto. The representative preparation examples will be described.

Preparation examples of a composition including a sample extract of the present disclosure are described, but the present disclosure is not limited thereto. The preparation examples are merely described in detail.

### Formulation example 1. Preparation of powder

| | |
|---|---|
| Extract (VSM) | 20 mg |
| Lactose | 100 mg |
| Talc | 10 mg |

The above components were mixed

### Formulation example 2. Preparation of tablets

| | |
|---|---|
| Fraction (VSW) | 10 mg |
| Corn starch | 100 mg |
| Lactose | 100 mg |
| Magnesium stearate | 2 mg |

The tablet was prepared by mixing the above components and en-tableting the same, according to an existing tablet formation method.

### Formulation example 3. Preparation of capsule

| | |
|---|---|
| Fraction (VS25E) | 10 mg |
| Crystalline cellulose | 3 mg |
| Lactose | 14.8 mg |
| Magnesium stearate | 2 mg |

The capsule was prepared by mixing the above components and filling the mixed components to a gelatin capsule, according to an existing capsule formation method.

### Formulation example 4. Preparation of injection

| | |
|---|---|
| Fraction (VS50E) | 10 mg |
| Mannitol | 180 mg |
| Sterilized distilled water for injection | 2974 mg |
| Na₂HPO₄,12H2O | 26 mg |

According to an existing injection formation method, the injection having the component content per one ampoule (2 mℓ) was prepared.

### Formulation example 5. Formation of liquid

| | |
|---|---|
| Extract (VS75E) | 20 mg |
| isomerized glucose syrup | 10 g |
| Mannitol | 5 g |
| Purified water | optimum amount |

According to an existing liquid preparation method, each component was added to the purified water and dissolved therein, and an optimum amount of lemon flavor was added and mixed with the above components. Then, purified water was added thereto, the total amount of components was adjusted to 100 mℓ, and 100 mℓ was filled in a brown bottle and sterilized, thereby preparing a liquid.

### Formulation example 6. Preparation of health food

| | |
|---|---|
| Extract (VSE) | 1000 mg |
| Vitamin mixture | optimum amount |
| Vitamin A acetate | 70 µg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 µg |
| Vitamin C | 10 mg |
| Biotin | 10 µg |
| nicotinic acid amide | 1.7 mg |
| Folic acid | 50 µg |
| calcium pantothenate | 0.5 mg |
| Mineral mixture | optimum amount |
| Ferrous sulfate | 1.75 mg |
| Zinc oxide | 0.82 mg |
| Magnesium carbonate | 25.3 mg |
| Monopotassium phosphate | 15 mg |
| Dicalcium phosphate | 55 mg |
| Potassium citrate | 90 mg |
| Calcium carbonate | 100 mg |
| Magnesium chloride | 24.8 mg |

A composition ratio of vitamins and a mineral mixture is a mixture of ingredients relatively suitable for health food, but a mixture ratio may be arbitrarily modified without departing from the spirit and scope of the present disclosure.

### Formation example 7. Preparation of a health beverage

| | |
|---|---|
| Extract (VSEA) | 1000 mg |
| Citric acid | 1000 mg |
| Oligosaccharide | 100 g |
| Apricot concentration | 2 g |
| Taurine | 1 g |
| Purified water | total 900 mℓ |

According to an existing health beverage preparation method, the above ingredients were mixed and then stirred at a temperature of about 85 °C for about one hour, and a generated solution was filtered and added to a sterilized 2L container. The container was sealed, sterilized, and stored in a refrigerator to be used to prepare of a health beverage composition.

The above composition ratio is a mixture of ingredients relatively suitable for favorite beverages, but the mixing ratio may be arbitrarily modified according to regional and ethnic preferences such as a demand class, a country of demand, and a purpose of use.

### INDUSTRIAL APPLICABILITY

As described above, the composition is useful as a pharmaceutical composition, health functional food, or health supplement food for prevention or treatment of inflammation, allergies, and asthma due to the confirmation, through various animal experiments, that a Veronicastrum sibiricum L. Pennell (genus Veronicastrum) extract exhibits excellent anti-inflammatory, anti-allergic and asthma-inhibitory activity, the animal experiments conducted using the Veronicastrum sibiricum L. Pennell (genus Veronicastrum) extract and compounds and including: evaluation on inhibition of leukotriene production (Experimental Example 1); effects on the total number of cells in bronchoalveolar lavage (BAL) fluid by using male Balb/c mice (Experimental Example 2); effects on the number of neutrophil cells relative to the total number of cells in the BAL fluid (Experimental Example 3); effects on the number of Neutrophil+/Gr-1+ absolute cells in the BAL fluid (Experimental Example 4); effects on the number of CD11b+/Gr-1+ absolute cells from among lung cells (Experimental Example 5); effects on the number of CD4+/CD3+ absolute cells from among lung cells (Experimental Example 6); effects on the number of Macrophage+/CD11b+ absolute cells from among lung cells (Experimental Example 7); effects on the expression of inflammation factors in the BAL fluid (Experimental Example 8).

## Claims

1. A pharmaceutical composition comprising a Veronicastrum sibiricum L. Pennell extract (genus Veronicastrum) as an active ingredient for use in the prevention or treatment of allergy and/or asthma.

2. The pharmaceutical composition for use according to claim 1, wherein Veronicastrum sibiricum L. Pennell comprises a root, a stem, or flowers from Korea, China, Russia, or Japan.

3. The pharmaceutical composition for use according to claim 1, wherein the Veronicastrum sibiricum L. Pennell extract comprises a crude extract, a polar soluble extract, or a non-polar soluble extract.

4. The pharmaceutical composition for use according to claim 3, wherein the crude extract comprises an extract soluble in water comprising purified water, a C₁ to C₄ lower alcohol such as methanol, ethanol, or butanol, or a mixture thereof.

5. The pharmaceutical composition for use according to claim 3, wherein the non-polar soluble extract comprises an extract soluble in hexane, methylene chloride, chloroform, or ethyl acetate.

6. The pharmaceutical composition for use according to claim 1, wherein the allergy is selected from the group consisting of hypersensitivity, allergic rhinitis, asthma, allergic conjunctivitis, allergic dermatitis, atopic dermatitis, contact dermatitis, hives, insect allergy, food allergy, and drug allergy.

7. The pharmaceutical composition for use according to et- claim 1, wherein the asthma comprises bronchial asthma caused by a factor selected from the group consisting of house dust mites, pollen, animal hair, dandruff, cockroaches, food, drugs, cigarette smoke, indoor pollution, air pollution, physical activity, yellow dust, and stress.

8. Health functional food comprising a Veronicastrum sibiricum L. Pennell (genus Veronicastrum) extract as an active ingredient for use in the prevention or improvement of allergy and/or asthma.

9. The health functional food for use according to claim 8, wherein the health functional food is in a form of powder, granules, tablets, capsules, pills, suspension, emulsion, syrup, tea bag, leached tea, or a health beverage.

10. A food additive comprising a Veronicastrum sibiricum L. Pennell (genus Veronicastrum) extract as an active ingredient for use in prevention or improvement of allergy and/or asthma.

## Patentansprüche

1. Pharmazeutische Zusammensetzung, die ein Extrakt aus Veronicastrum Sibiricum L. Pennell (Gattung Veronicastrum) als einen aktiven Inhaltsstoff enthält, für die Verwendung bei der Vorbeugung oder Behandlung von Allergien und/oder Asthma.

2. Pharmazeutische Zusammensetzung für die Verwendung nach Anspruch 1, wobei Veronicastrum Sibiricum L. Pennell eine Wurzel, einen Stiel oder Blüten aus Korea, China, Russland oder Japan umfasst.

3. Pharmazeutische Zusammensetzung für die Verwendung nach Anspruch 1, wobei der Extrakt aus Veronicastrum Sibiricum L. Pennell einen Rohextrakt, einen polaren löslichen Extrakt oder einen nicht polaren löslichen Extrakt umfasst.

4. Pharmazeutische Zusammensetzung für die Verwendung nach Anspruch 3, wobei der Rohextrakt einen Extrakt umfasst, der in Wasser, das gereinigtes Wasser, einen C₁ bis C₄ niedrigeren Alkohol, wie etwa Methanol, Äthanol oder Butanol oder eine Mischung davon umfasst, löslich ist.

5. Pharmazeutische Zusammensetzung für die Verwendung nach Anspruch 3, wobei der nicht polare lösliche Extrakt einen Extrakt umfasst, der in Hexan, Methylenchlorid, Chloroform oder Ethylacetat löslich ist.

6. Pharmazeutische Zusammensetzung für die Verwendung nach Anspruch 1, wobei die Allergie aus der Gruppe gewählt ist, die aus einer Überempfindlichkeit, allergischer Rhinitis, Asthma, allergischer Bindehautentzündung, allergischer Dermatitis, atopischer Dermatitis, Kontaktdermatitis, Nesselsucht, Insektenallergie, Lebensmittelallergie und Arzneimittelallergie besteht.

7. Pharmazeutische Zusammensetzung für die Verwendung nach Anspruch 1, wobei das Asthma Bronchialasthma umfasst, das durch einen Umstand ausgelöst wird, der aus der Gruppe ausgewählt ist, die aus Hausstaubmilben, Pollen, Tierhaaren, Schuppen, Kakerlaken, Lebensmitteln, Medikamenten, Zigarettenrauch, Innenraumverschmutzung, Luftverschmutzung, physischer Aktivität, gelbem Staub und Stress besteht.

8. Gesundheitsfunktionsnahrung, die einen Extrakt aus Veronicastrum Sibiricum L. Pennell (Gattung Veronicastrum) als einen aktiven Inhaltsstoff enthält, für die Verwendung bei der Vorbeugung oder Behandlung von Allergien und/oder Asthma.

9. Gesundheitsfunktionsnahrung für die Verwendung nach Anspruch 8, wobei die Gesundheitsfunktionsnahrung die Form eines Pulvers, eines Granulats, von Tabletten, von Kapseln, von Pillen, einer Suspension, einer Emulsion, eines Sirups, einem Teebeutel, von ausgelaugtem Tee oder eines Gesundheitsgetränks hat.

10. Lebensmittelzusatz, der einen Extrakt aus Veronicastrum Sibiricum L. Pennell (Gattung Veronicastrum) als einen aktiven Inhaltsstoff enthält, für die Verwendung bei der Vorbeugung oder Behandlung von Allergien und/oder Asthma.

## Revendications

1. Composition pharmaceutique comportant un extrait de Veronicastrum sibiricum L. Pennell (genre Veronicastrum) en tant qu'ingrédient actif pour une utilisation dans la prévention ou le traitement d'une allergie et/ou d'un asthme.

2. Composition pharmaceutique pour une utilisation selon la revendication 1, dans laquelle Veronicastrum sibiricum L. Pennell comporte une racine, une tige ou des fleurs de Corée, Chine, Russie ou Japon.

3. Composition pharmaceutique pour une utilisation selon la revendication 1, dans laquelle l'extrait de Veronicastrum sibiricum L. Pennell comporte un extrait brut, un extrait soluble polaire ou un extrait soluble apolaire.

4. Composition pharmaceutique pour une utilisation selon la revendication 3, dans laquelle l'extrait brut comporte un extrait soluble dans l'eau comportant de l'eau purifiée, un alcool de faible poids moléculaire en C₁ à C₄ tel que le méthanol, l'éthanol ou le butanol, ou un mélange de ceux-ci.

5. Composition pharmaceutique pour une utilisation selon la revendication 3, dans laquelle l'extrait soluble non polaire comporte un extrait soluble dans l'hexane, le chlorure de méthylène, le chloroforme ou de l'acétate d'éthyle.

6. Composition pharmaceutique pour une utilisation selon la revendication 1, dans laquelle l'allergie est choisie parmi le groupe constitué d'une hypersensibilité, d'une rhinite allergique, d'un asthme, d'une conjonctivite allergique, d'une dermatite allergique, d'une dermatite atopique, d'une dermatite de contact, d'un urticaire, d'une allergie aux insectes, d'une allergie alimentaire et d'une allergie médicamenteuse.

7. Composition pharmaceutique pour une utilisation selon la revendication 1, dans laquelle l'asthme comporte un asthme bronchique causé par un facteur choisi parmi le groupe constitué des acariens de la poussière, du pollen, des poils d'animaux, des pellicules, des cafards, des aliments, des médicaments, de la fumée de cigarette, de la pollution intérieure, de la pollution de l'air, de l'activité physique, de la poussière jaune et du stress.

8. Aliment fonctionnel naturel comportant un extrait de Veronicastrum sibiricum L. Pennell (genre Veronicastrum) en tant qu'ingrédient actif pour une utilisation dans la prévention ou l'amélioration d'une allergie et/ou d'un asthme.

9. Aliment fonctionnel naturel pour une utilisation selon la revendication 8, dans lequel l'aliment fonctionnel naturel se présente sous forme de poudre, de granulés, de comprimés, de capsules, de pilules, de suspension, d'émulsion, de sirop, de sachet de thé, de thé filtré ou de boisson diététique.

10. Additif alimentaire comportant un extrait de Veronicastrum sibiricum L. Pennell (genre Veronicastrum) en tant qu'ingrédient actif pour une utilisation dans la prévention ou l'amélioration d'une allergie et/ou d'un asthme.
